# EUROPÄISCHE PATENTSCHRIFT

(11) **EP 1 610 690 B1**
(45) Veröffentlichungstag und Bekanntmachung des Hinweises auf die Patenterteilung: **19.05.2010**
(21) Anmeldenummer: 04723997.5
(22) Anmeldetag: 29.03.2004
(51) Int. Cl.: A61B 10/00

(54) **DRUCKERZEUGUNGSEINHEIT**
PRESSURE GENERATING UNIT
UNITE DE GENERATION DE PRESSION

(30) Priorität: 29.03.2003 DE 10314240
(43) Veröffentlichungstag der Anmeldung: 04.01.2006
(73) Patentinhaber: C.R. BARD, INC., Murray Hill, NJ 07974 (US)
(72) Erfinder: HESKE, Norbert, F., 82288 Kottgeisering (DE); HESKE, Thomas, 82284 Grafrath (DE)
(74) Vertreter: Tomlinson, Edward James
(86) Internationale Anmeldenummer: PCT/EP2004/003328
(87) Internationale Veröffentlichungsnummer: WO 2004/086978

(56) Entgegenhaltungen:
- EP-A- 1 074 271
- WO-A-96/28097
- DE-U- 20 204 363
- US-A- 5 971 939

## Beschreibung

Die Erfindung betrifft eine Druckerzeugungseinheit, insbesondere eine Druckerzeugungseinheit, die im Handstück einer Vakuum-Biopsievorrichtung angeordnet ist, die in Art einer Spritze ausgebildet ist und wobei durch Zurückziehen des Kolbens im evakuierten Zylinderraum bei der Umstellung auf die Erzeugung von Überdruck die Luftzufuhr durch die Position des Kolbens freigegeben wird.

Eine derartige Druckerzeugungseinheit in einer Vakuum-Biopsievorrichtung ist aus der DE 202 04363 U oder der DE 20211934 U bekannt. Die Druckerzeugungseinheit ist über eine Verbindungsleitung mit einer Biopsienadeleinheit verbunden, die in das zu untersuchende Gewebe eingeschossen wird. Die Druckerzeugungseinheit und die Nadeleinheit sind parallel in dem Gehäuse eines Handstücks angeordnet. Zur Entnahme der Probe wird Vakuum mittels der Druckerzeugungseinheit im Nadelraum erzeugt.

Um die Druckerzeugungseinheit auch zum Erzeugen eines Überdrucks verwenden zu können, ist eine Belüftungsöffnung vorgesehen, bei deren Freigabe durch den Spritzenkolben das erzeugte Vakuum abgebaut wird, so dass die dann eingedrungende Luft mittels des Spritzenkolbens komprimiert werden kann.

Da durch das Vakuum nicht nur die Probe in den Probeentnahmeraum eingesogen wird, sondern auch Gewebeflüssigkeit, kann insbesondere bei ungünstiger Lage der Druckerzeugungseinheit, bzw. des Handstücks mit Nadeleinheit und der damit verbundenen Druckerzeugungseinheit bei der kurzzeitigen Freigabe der Belüftungsöffnung Gewebeflüssigkeit, die in den Zylinderkolbenraum eingedrungen ist, in den Innenraum des Handstücks ausfließen. Um dies zu verhindern, ist schon vorgeschlagen worden, die Belüftungsöffnung mittels eines Schwammes, der auf der Außenseite des Kolbenzylinders angeordnet ist abzudichten. Dies ist jedoch nicht in allen Fällen ausreichend.

Eine Druckerzeugungseinheit gemäß der Präambel von Anspruch 1 ist aus der US 5,971,939 bekannt.

Aufgabe der vorliegenden Erfindung ist daher einerseits die für den Abbau des Vakuums nötige Luft in den Zylinder einströmen zu lassen und andererseits eine Verschmutzung des Gehäuseinnenraums des Handstücks durch ausfließende Gewebeflüssigkeit zuverlässig zu verhindern.

Die Lösung der Aufgabe wird gemäß Anspruch 1 darin gesehen, dass der evakuierte Zylinderraum über eine Verbindungsleitung mit dem unter Atmosphärendruck stehenden Zylinderraum auf der anderen Kolbenseite verbunden ist und auf der Kolbenspindel ein saugfähiges Vlies angeordnet ist.

Durch die Anordnung einer Verbindung zwischen den beiden durch den Kolben unterteilten Zylinderräumen, wobei die Verbindung durch die Stellung des Kolbens freigegeben oder verschlossen wird, und die Anordnung eines saugfähigen Vlies im unter Atmosphärendruck stehenden Zylinderraum kann einerseits Luft von Außen in den evakuierten Teil des Zylinders einströmen und andererseits wird durch das saugfähige Vlies ausströmende Gewebeflüssigkeit aus dem evakuierten Zylinderteil aufgesaugt. Die Länge der Verbindung ist so gewählt, dass die Nut nach der Freigabe der Verbindung für das Einströmen von Luft, die im unter Atmosphärendruck endende Öffnung des Zylinderraums über dem saugfähigen Vlies liegt. Dies hat den Vorteil, dass die beim kurzzeitigen Öffnen der Verbindung evtl. austretende Gewebeflüssigkeit unmittelbar in das Vlies geleitet und von diesem aufgesaugt wird.
Die Verwendung eines luftdurchlässigen, saugfähigen Vlies hat darüber hinaus den Vorteil, dass die eintretende Luft gefiltert wird und somit Partikel nicht in den Zylinderraum gelangen.

Als Material für das Vlies hat sich als besonders einfach und preisgünstig die Verwendung von Zellstoff, insbesondere saugfähiges Papier herausgestellt. Vorteilhafterweise ist das Vlies mittels einer auf der Kolbenspindel angeordneten Sicherungsscheibe unverschiebbar gehalten. Dies hat den Vorteil, dass im Betrieb das Vlies nicht auf der Kolbenspindel wandern kann und dadurch die Wirksamkeit des Vlies vermindert wird.

Nachstehend soll die Erfindung anhand eines Ausführungsbeispiels näher erläutert werden. Es zeigt:
Fig. 1) Die Biopsievorrichtung
Fig. 2) Die Druckerzeugungseinheit mit eingefahrenem Kolben (teilweise geschnitten)
Fig. 3) Die Druckerzeugungseinheit nach dem Erzeugen eines Vakuums durch Zurückziehen des Kolbens
Fig. 4) Die Druckerzeugungseinheit nach Freigabe der Verbindung für die Belüftung
Fig. 5) Schnitt A - A durch Fig. 4
Fig. 6) Schnitt B - B durch Fig. 5

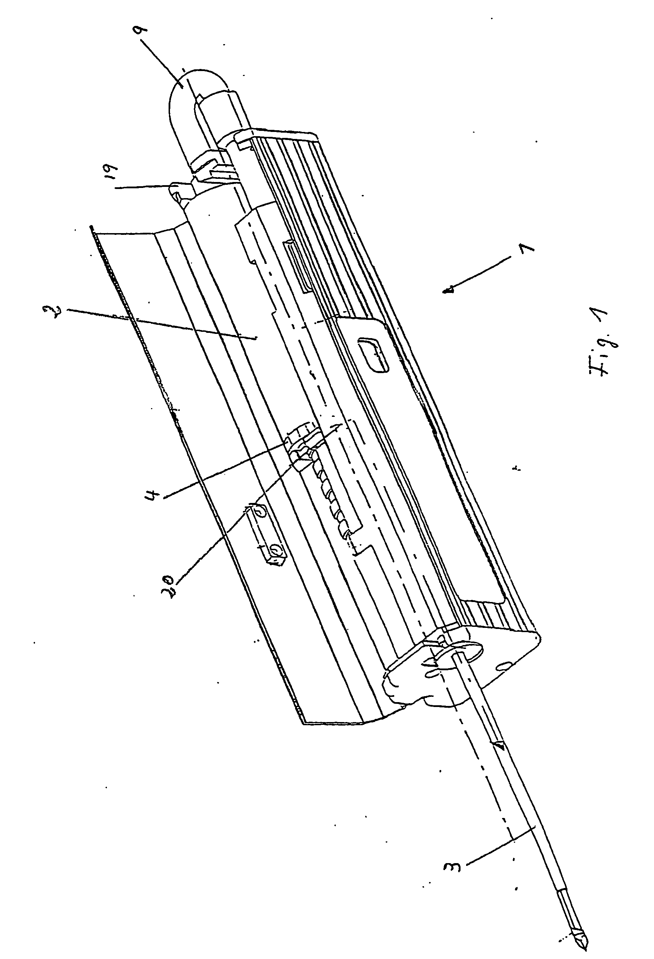

Fig. 1 zeigt eine Biopsievorrichtung 1, bei der die Druckerzeugungseinheit 2 mit einer parallel liegenden Nadeleinheit 3 in einem Gehäuse untergebracht ist. Die Druckerzeugungseinrichtung wird z.B. über einen Elektrogetriebemotor (nicht dargestellt) über das Zahnrad 4 angetrieben.

Die Druckerzeugungseinheit 2 , die in Art einer Spritze aufgebaut ist, besteht aus einem Zylinder 5, in dem ein Kolben 6 mittels einer Kolbenspindel 7 längsverschieblich bewegbar ist. Der Kolbenspindelantrieb besteht aus einem am offenen Ende des Zylinders gelagerten Zahnrad 4, wobei das Zentrum des Zahnrads als Spindelmutter ausgebildet ist, das mit der darin gelagerten Kolbenspindel 7 zusammenwirkt. Über ein nicht dargestelltes Ritzel, das auf der Welle eines Elektromotors sitzt wird die Kolbenspindel 7 mittels des Zahnrads 4 je nach Drehrichtung des Motors zum Anschluss 8 oder zum Zahnrad 4 hin bewegt. Der Zylinder der Druckerzeugungseinheit weist an einem Ende einen Anschluss 8 für ein Verbindungsstück 9 auf, das mit der Biopsienadeleinheit 3 verbunden ist. Auf der dem Anschluss entgegengesetzten Seite ist ein Zahnrad 4 mit innen liegendem Spindelgewinde (Spindelmutter) angeordnet, das mit der Kolbenspindel 7 zusammenwirkt, so dass der Kolben 6 bei jeder Umdrehung des Zahnrads einen genau definierten Weg, je nach Motordrehung, nach der einen oder anderen Seite zurücklegt. Das Zahnrad kann im offenen Zylinderende gelagert sein.

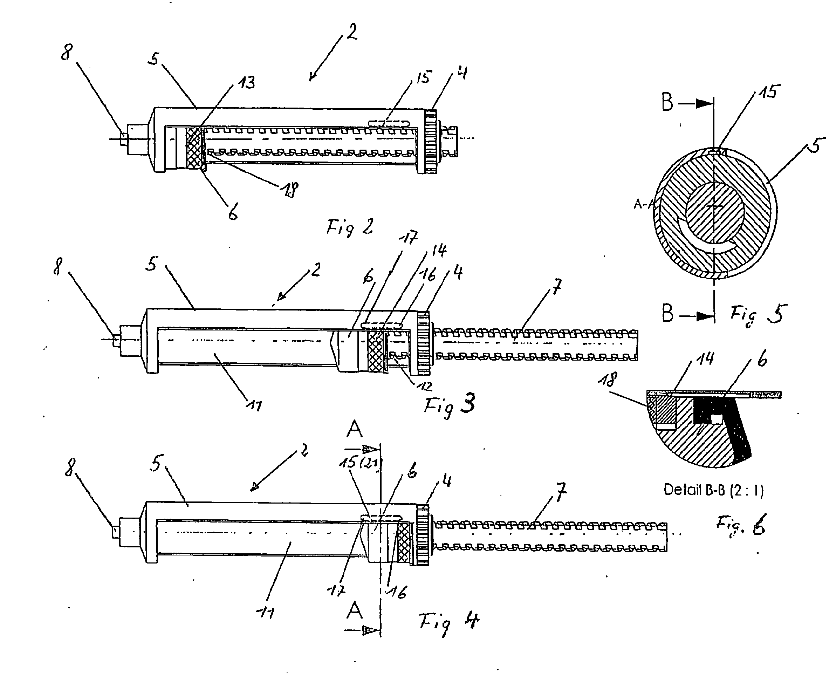

Je nach Drehrichtung kann also der Kolben 6 über den Zahnrad-/Spindelantrieb zum Zylinderboden hin oder vom Zylinderboden weg zum Zahnrad hin bewegt werden. Die Druckerzeugungseinheit ist in einer Biopsievorrichtung wie sie Fig. 1 zeigt und in der DE 202 04 363 U näher beschrieben ist, beispielsweise eingebaut; der Abstand zwischen Gehäusewand 19 und Einlegenut 20 für die Kolbenspindel ist so gewählt, dass die Druckerzeugungseinheit sich nicht in der Längsache verschieben kann und das Zahnrad 4 damit im Zylinder abgestützt ist. Beim Zurückfahren des Kolbens bis kurz vor die Öffnung der Verbindungsleitung 21, hier einer Nut 15 in der Zylinderwand, also in Richtung Zahnrad 4, wird im Biopsienadelsystem ein Vakuum aufgebaut (s. Fig 3). Nach Freigabe der Luftzufuhr im Zylinderraum 11 (Öffnung der Verbindungsleitung, Nut ist geöffnet) - wie nachstehend beschrieben - wird in dem Biopsienadelsystem der vorher aufgebaute Unterdruck (s. Fig. 4) durch das Einströmen von Luft abgebaut. Wird der Kolben nach dem Einströmen der Luft in Richtung Anschluss 8 bewegt, so wird im System Überdruck erzeugt.

Die Kolbenspindel trägt auf der dem Antrieb entgegengesetzten Seite, also auf der Anschlussseite, den Kolben 6 mit Gummimantel. Der Gummimantel des Kolbens dichtet an der Kolbenzylinderinnenwand den linken Zylinderraum 11 (Raum vor dem Anschluß) vom Zylinderraum 12 ab. Ist also der Anschlussstutzen 8 über das Verbindungsstück 9 mit der Biopsienadeleinheit verbunden und die Biopsienadel z.B. in ein Gewebe eingebracht, so entsteht durch die Verschiebung des Kolbens zur Antriebsseite ein Unterdruck im Biopsienadelsystem. Der Zylinderraum 12 steht weiterhin unter Atmosphärendruck. An der Seitenwand 13 des Kolbens, die im Zylinderraum 12 liegt, ist ein saugfähiges Vlies 14 angeordnet, das von der Kolbenspindel koaxial durchdrungen wird und das z.B. mittels einer Sicherungsscheibe 18, die auf der Kolbenspindel befestigt ist, gehalten wird. Das Vlies ist rund ausgebildet und liegt leicht dichtend an der Innenwand des Zylinders an. Um es leicht über die Kolbenspindel stülpen zu können ist das als Lochscheibe ausgebildete Vlies geschlitzt. Das Vlies kann aus mehreren einzelnen Scheiben von ca. 1mm Stärke bestehen. Es kann aber auch einteilig sein. Es hat eine Erstreckung von ca. 3mm. Das Vlies ist unmittelbar auf der Kolbenseitenwand 13 aufgesetzt und wird durch die Sicherungsscheibe gehalten. An dem dem Zahnrad 4 benachbarten Zylinderteil ist als Verbindung 21, eine Nut 15, in die Innenwand der Zylinderwand eingearbeitet. Wie Fig. 5 zeigt, entspricht die Nuttiefe etwa der Hälfte der Wandstärke. Die Nutlänge (Fig. 5) ist so gewählt, dass die Nut bei Freigabe der Luftzufuhr in der Mitte des saugfähigen Vlies 14 endet und über die Nut der zu belüftende Zylinderraum 11 mit dem außen liegenden Atmosphärendruck verbunden ist. Die Nut hat in dieser Stellung gewissermaßen zwei "Öffnungen". Die eine "Öffnung 17" endet im Zylinderraum 11, die andere "Öffnung 16" endet über dem Fließ 14 wenn der Kolben in Öffnungsstellung gebracht wurde (sh. Fig. 4).

Bei Einsatz des Vakuum-Biopsiegerätes nach der DE 202 04 363 U oder DE 202 11 934 U hat sich gezeigt, dass die Sogwirkung der Druckerzeugungseinheit 2 so stark ist, dass je nach Lage der Biopsievorrichtung bei der Probenentnahme mehr oder weniger Gewebeflüssigkeit in die Druckerzeugungseinheit 2 gelangen kann. Durch die Anordnung einer Nut 15 im Innenraum des Zylinders, die vor allem wegen des Abbaus des Unterdrucks erforderlich ist, kann der Ausfluss von Gewebeflüssigkeit, bei der kurzzeitigen Öffnung der Belüftungsöffnung und dem anschließenden Verschließen nicht immer vermieden werden.
Da aber die Nut so gestaltet ist, dass die "Öffnung 16" über dem saugfähigen Vlies endet, wird die Gewebeflüssigkeit aufgesaugt und es fließt keine Gewebeflüssigkeit in das Gehäuse des Biopsiehandstücks. Bei der Freigabe der "Öffnung 16" der Nut (s. Fig. 4) kann die Luft, die über den Zylinderraum 9 über das saugfähige Vlies über die Nut in den Zylinderraum 11 gelangen und dort das Vakuum abbauen. Die Luft wird also vor ihrem Eintritt in den Zylinderraum 11 gefiltert. Das Einfließen von Gewebeflüssigkeit in das Gehäuse des Handstücks des Biopsiegerätes ist wegen der eingebauten elektronischen Bestandteile unbedingt zu vermeiden, da eine Nassreinigung des Handstücks zu großen Schäden an der Elektronik führen kann.
Im Ausführungsbeispiel ist als Verbindung vom Zylinderraum 11 mit dem Zylinderraum 12 eine innen liegende Nut vorgesehen. Die Verbindung kann auch als außen liegende Leitung oder eine in den Zylindermantel integrierte Leitung ausgebildet sein. Wichtig für die Lösung des Problems ist, dass die Gewebeflüssigkeit, die beim Abbau des Vakuums austreten kann, gezielt so geleitet wird, dass die Gewebeflüssigkeit mittels eines saugfähigen Fließ aufgesaugt wird und nicht in das Gehäuse gelangt.

### Teileliste

- 1): Biopsievorrichtung
- 2): Druckerzeugungseinheit
- 3): Nadeleinheit
- 4): Zahnrad
- 5): Zylinder
- 6): Kolben
- 7): Kolbenspindel
- 8): Anschluss
- 9): Verbindungsstück
- 10) 11): Zylinderraum
- 12): Zylinderraum
- 13): Seitenwand
- 14): saugfähiges Vlies
- 15): Nut
- 16): Öffnung
- 17): Öffnung
- 18): Sicherungsscheibe
- 19): Gehäusewand
- 20): Einlegenut
- 21): Verbindungsleitung
- 22):
- 23):
- 24):
- 25):
- 26):
- 27):
- 28):
- 39):
- 39:

## Patentansprüche

1. Druckerzeugungseinheit, insbesondere Druckerzeugungseinheit, die im Handstück einer Vakuum-Biopsievorrichtung angeordnet ist, wobei die Druckerzeugungseinheit in Art einer Spritze ausgebildet ist, einen Zylinder (5), einen Kolben (6) und eine Kolbenspindel (7) enthält und wobei die Luftzufuhr inden durch Zurückziehen des Kolbens evakuierten Zylinderraum bei der Umstellung auf die Erzeugung von Überdruck durch eine Freigabeposition des Kolbens freigegeben wird, **dadurch gekennzeichnet, dass** der evakuierte Zylinderraum (11) in der Freigabeposition über eine Verbindungsleitung (21) mit dem unter Atmosphärendruck stehenden Zylinderraum (12) auf der Kolbenrückseite verbunden ist und auf der Kolbenspindel (7) ein saugfähiges Fließ (14) angeordnet ist, so dass beim kurz zeitigen Öffnen der Verbindungsleitung austretende Gewebeflüssigkeit unmittelbar in das Vlies geleitet und von diesem aufgesaugt wird.

2. Druckerzeugungseinheit nach Anspruch 1 **dadurch gekennzeichnet, dass** die Verbindungsleitung als innen liegende Nut (15) im Zylinder ausgebildet ist, die bei nach der Antriebsseite zurückgefahrenem Kolben eine Verbindung zwischen evakuiertem Zylinderraum (11) und dem mit Atmosphärenluft verbundenen Zylinderraum (12) herstellt, und dass das saugfähige Vlies (14) auf der Kolbenspindel zwischen Kolbenrückseite und Zylinder innenwand in dem Zylinderraum (12) angeordnet ist.

3. Druckerzeugungseinheit nach Anspruch 2 **dadurch gekennzeichnet, dass** die Nut (15) nach dem Öffnen der Verbindung zwischen den beiden Zylinderräumen (11,12) über dem Vlies (14) endet.

4. Druckerzeugungseinheit nach Anspruch 1, **dadurch gekennzeichnet, dass** das Vlies (14) aus saugfähigem Zellstoff besteht.

5. Druckerzeugungseinheit nach Anspruch 2, **dadurch gekennzeichnet, dass** ein Papierfilter als Vlies (14) verwendet wird.

6. Druckerzeugungseinheit nach einem der vorhergehenden Ansprüche, **dadurch gekennzeichnet, dass** das Vlies (14) auf der Kolbenspindel (7) mit einer auf der Kolbenspindel angeordneten Sicherungsscheibe (18) in ihrer Position gehalten wird.

7. Druckerzeugungseinheit nach Anspruch 6, **dadurch gekennzeichnet, dass** das Vlies (14) dicht auf der Kolbenrückseite angeordnet ist und durch die Sicherungsscheibe (18) gehalten wird.

## Claims

1. Pressure generating unit, in particular a pressure generating unit arranged in the handpiece of a vacuum biopsy device, the pressure generating unit being designed in the form of a syringe and containing a cylinder (5), a piston (6) and a piston spindle (7), and the air supply in the cylinder chamber evacuated by drawing back the piston being released by a release position of the piston during the switchover to generating positive pressure, **characterized in that** the evacuated cylinder chamber (11) in the release position is, by means of a connection line (21), connected to the cylinder chamber (12), which is under atmospheric pressure and situated on the rear side of the piston, and an absorbent nonwoven fabric (14) is arranged on the piston spindle (7), and therefore tissue liquid emerging from the connection line when the latter is opened briefly is guided directly onto the nonwoven fabric and absorbed thereby.

2. Pressure generating unit according to Claim 1, **characterized in that** the connection line is designed as a groove (15) lying on the inside in the cylinder and a connection between the evacuated cylinder chamber (11) and the cylinder chamber (12) connected to the atmosphere is produced by said groove when the piston is retracted to the drive side, and **in that** the absorbent nonwoven fabric (14) is arranged in the cylinder chamber (12), on the piston spindle between the rear side of the piston and the inner wall of the cylinder.

3. Pressure generating unit according to Claim 2, **characterized in that** the groove (15) terminates above the nonwoven fabric (14) after opening the connection between the two cylinder chambers (11, 12).

4. Pressure generating unit according to Claim 1, **characterized in that** the nonwoven fabric (14) consists of absorbent pulp.

5. Pressure generating unit according to Claim 2, **characterized in that** a paper filter is used as a nonwoven fabric (14).

6. Pressure generating unit according to one of the preceding claims, **characterized in that** the nonwoven fabric (14) is held in position on the piston spindle (7) by a securing disc (18) arranged on the piston spindle.

7. Pressure generating unit according to Claim 6, **characterized in that** the nonwoven fabric (14) is arranged closely on the rear side of the piston and is held by the securing disc (18).

## Revendications

1. Unité de génération de pression, en particulier unité de génération de pression qui est disposée dans la poignée d'un dispositif de biopsie à vide, l'unité de génération de pression étant réalisée sous forme de seringue, comprenant un cylindre (5), un piston (6) et une tige de piston (7), et l'alimentation en air dans l'espace cylindrique évacué en retirant le piston étant libérée lors du passage à la génération de surpression par une position de libération du piston, **caractérisée en ce que** l'espace cylindrique (11) évacué est connecté dans la position de libération par le biais d'une conduite de connexion (21) à l'espace cylindrique (12) à pression atmosphérique au niveau du côté arrière du piston, et un voile aspirant (14) étant disposé sur la tige du piston (7), de sorte que dans le cas d'une ouverture de courte durée de la conduite de connexion, du liquide sortant provenant des tissus soit immédiatement conduit dans le voile et soit aspiré par celui-ci.

2. Unité de génération de pression selon la revendication 1, **caractérisée en ce que** la conduite de connexion est réalisée sous forme de rainure intérieure (15) dans le cylindre, laquelle, lorsque le piston a été ramené en arrière vers le côté d'entraînement, crée une connexion entre l'espace cylindrique évacué (11) et l'espace cylindrique (12) connecté à l'air de l'atmosphère, et **en ce que** le voile aspirant (14) est disposé dans l'espace cylindrique (12) sur la tige de piston entre le côté arrière du piston et la paroi intérieure du cylindre.

3. Unité de génération de pression selon la revendication 2, **caractérisée en ce que** la rainure (15) se termine après l'ouverture de la connexion entre les deux espaces cylindriques (11, 12) au-dessus du voile (14).

4. Unité de génération de pression selon la revendication 1, **caractérisée en ce que** le voile (14) se compose d'une cellulose aspirante.

5. Unité de génération de pression selon la revendication 2, **caractérisée en ce que** l'on utilise comme voile (14) un filtre en papier.

6. Unité de génération de pression selon l'une quelconque des revendications précédentes, **caractérisée en ce que** le voile (14) est maintenu dans sa position sur la tige de piston (7) par un disque de fixation (18) disposé sur la tige de piston.

7. Unité de génération de pression selon la revendication 6, **caractérisée en ce que** le voile (14) est disposé hermétiquement sur le côté arrière du piston et est maintenu par le disque de fixation (18).
